(19) Europäisches Patentamt
European Patent Office
Office européen des brevets

(11) Veröffentlichungsnummer: **0 606 863 A2**

# (12) EUROPÄISCHE PATENTANMELDUNG

(21) Anmeldenummer: **94100220.6**

(51) Int. Cl.5: **C07C 41/46**, C09K 15/18

(22) Anmeldetag: **08.01.94**

(30) Priorität: **15.01.93 DE 4300892**

(43) Veröffentlichungstag der Anmeldung:
**20.07.94 Patentblatt 94/29**

(84) Benannte Vertragsstaaten:
**AT BE DE FR GB IT NL**

(71) Anmelder: **HOECHST AKTIENGESELLSCHAFT**

**D-65926 Frankfurt(DE)**

(72) Erfinder: **Glass, Brunhilde**
**Weidenstrasse 10 a**
**D-84489 Burghausen(DE)**

## (54) Verfahren zur thermooxidativen Stabilisierung von Glykolverbindungen und thermooxidativ stabilisierte Mischungen davon.

(57) Nach dem beschriebenen Verfahren werden Glykolverbindungen organische Stickstoffverbindungen der Formel (1),

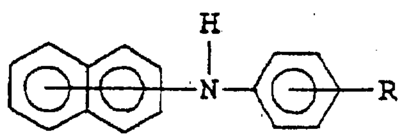

(1)

worin R der nachstehende Alkylrest ist,

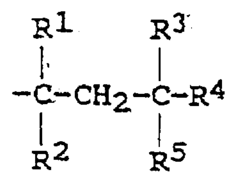

worin $R^1$ bis $R^5$ ein $C_1$ bis $C_4$-Alkyl ist, einverleibt, die eine hohe und lang anhaltende thermooxidative Stabilität bewirken. Die beschriebenen Mischungen bestehen im wesentlichen aus mindestens einer Glykolverbindung als Hauptkomponente und mindestens einer der angegebenen organischen Stickstoffverbindungen als thermooxidativer Stabilisator.

EP 0 606 863 A2

Die Erfindung betrifft ein Verfahren zur thermooxidativen Stabilisierung von Glykolverbindungen aus der Gruppe der Oxalkylenglykole und deren Mono- und Dialkylether. Die Erfindung betrifft ferner eine Mischung bestehend im wesentlichen aus mindestens einer dieser Glykolverbindungen und einem gegen thermooxidativen Abbau wirkenden Stabilisierungsmittel.

Oxalkylenglykole und deren Alkylether stellen bekanntlich vorteilhafte Lösungs-, Reinigungs- und Verdünnungsmittel dar. In dieser Funktion werden sie beispielsweise zur Herstellung von Lacken, Holzbeizen, Klebstoffen und hydraulischen Flüssigkeiten eingesetzt sowie zur Entfernung von sauren Gasen oder Lösemitteln aus entsprechenden Gasströmen.

Es ist ferner bekannt, daß die genannten Glykolverbindungen in Gegenwart von oxidierenden Komponenten wie Sauerstoff oder Luft Peroxide bilden, aus denen durch weiteren Abbau verfärbend und/oder korrosiv wirkende Zersetzungsprodukte entstehen wie Aldehyde, Ester und Säuren, und daß die Bildung von Peroxiden und deren Abbau durch Wärme gefördert und beschleunigt wird.

Gleichzeitige Anwesenheit von Luft und Wärme liegt insbesondere vor bei der obenerwähnten Entfernung von sauren Komponenten wie Schwefeldioxid, Schwefelwasserstoff und Kohlendioxid oder von Lösemitteln wie Kohlenwasserstoffe, halogenierte Kohlenwasserstoffe, Aromaten, Alkohole, Ketone, Ether und Ester aus entsprechenden Gasen. Die Reinigung solcher Gase erfolgt kurz zusammengefaßt in der Weise, daß man das Gas, auch Rauchgas oder Abgas genannt, durch vorzugsweise Oxalkylenglykoldialkylether leitet, wobei die genannten sauren Komponenten und Lösemittel absorbiert oder herausgelöst werden. Zur Desorption wird der beladene Dialkylether in der Regel bei einer Temperatur von bis zu etwa 150 °C gestrippt oder einer Destillation unterworfen. Bei diesen Verfahren ist die Gegenwart von Luft in der Regel nicht zu vermeiden. Hier wirken also hohe Temperatur und Luft als oxidierendes Medium gleichzeitig auf die eingesetzten Oxalkylenglykolverbindungen ein. Dazu kommt noch, daß häufig katalytisch wirkende Substanzen, beispielsweise Schwefelsäure, anwesend sind, was den obenerwähnten Abbau unter Umständen noch fördert.

Es sind bereits zahlreiche als Antioxidantien bezeichnete organische Verbindungen bekannt, deren Einsatz überall dort empfohlen wird, wo mit Peroxidbildung oder anderen Oxidationsreaktionen zu rechnen ist. Sie werden auch zur Stabilisierung der genannten Glykolverbindungen eingesetzt. In der europäischen Patentschrift 0 289 935 B1 ist ein Gemisch aus mindestens einer organischen Schwefelverbindung und mindestens einer organischen Stickstoffverbindung als thermooxidativer Stabilisator für Oxalkylenglykolalkylether beschrieben, dessen Wirkung aber noch zu wünschen übrig läßt.

Die Aufgabe der Erfindung besteht demnach darin, ein Stabilisierungsmittel für Glykolverbindungen der genannten Art vorzuschlagen, insbesondere für Oxalkylenglykolalkylether, das diesen Verbindungen eine hohe thermooxidative Stabilität verleiht.

Das erfindungsgemäße Verfahren zur thermooxidativen Stabilisierung von Glykolverbindungen der genannten Art ist dadurch gekennzeichnet, daß den Glykolverbindungen mindestens eine organische Stickstoffverbindung der nachstehenden Formel (1)

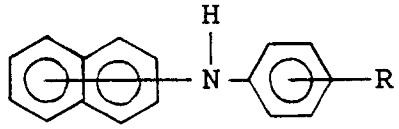 (1)

worin R der nachstehende Alkylrest ist

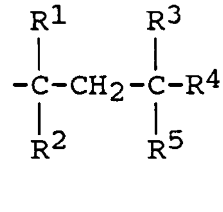

worin $R^1$ bis $R^5$, gleich oder verschieden,
vorzugsweise gleich, ein $C_1$ bis $C_4$-Alkyl ist,
vorzugsweise Methyl oder Ethyl,
in einer wirksamen Menge einverleibt wird.

Beim erfindungsgemäßen Verfahren werden also die angegebenen organischen Stickstoffverbindungen (die im Handel erhältlich sind) den zu stabilisierenden Oxalkylenglykolen und/oder deren Alkylethern in einer wirksamen Menge zugesetzt oder eingemischt. Sie sind im allgemeinen gut löslich. Das Zusetzen, Einmischen oder Einverleiben, gegebenenfalls unter Rühren, wird im allgemeinen bei Raumtemperatur (das sind etwa 15 bis 25 °C) durchgeführt. Die stabilisierende Wirkung wird durch Zusatz von mindestens einer der Stickstoffverbindungen erreicht, es können auch zwei oder mehr Stickstoffverbindungen zugesetzt werden. Die für eine gute Stabilisierung erforderliche Menge an Stabilisierungsmittel kann in weiten Grenzen variieren und hängt vor allem von der Temperatur und der Anwesenheit von Luft oder Sauerstoff und/oder von chemisch oder katalytisch aktiven Komponenten ab. Sie beträgt im allgemeinen 0,05 bis 10 Gew.-%, vorzugsweise 0,1 bis 5 Gew.-%, Gewichtsprozente bezogen auf die stabilisierte Mischung.

Die erfindungsgemäße, gegen oxidativen und/oder thermischen Abbau stabilisierte Mischung besteht demnach im wesentlichen aus mindestens einer Glykolverbindung der genannten Art als Hauptkomponente oder Hauptmenge und mindestens einer organischen Stickstoffverbindung der angegebenen Formel (1) in einer wirksamen Menge, wobei die wirksame Menge im allgemeinen 0,05 bis 10 Gew.-% beträgt, vorzugsweise 0,1 bis 5 Gew.-%, bezogen auf die Mischung. Anders ausgedrückt besteht die erfindungsgemäße Mischung im wesentlichen aus 0,05 bis 10 Gew.-%, vorzugsweise 0,1 bis 5 Gew.-%, von mindestens einer organischen Stickstoffverbindung der Formel (1) und mindestens einer Glykolverbindung der genannten Art, die im wesentlichen den Rest auf 100 Gew.-% darstellt, Gewichtsprozente bezogen auf das Gewicht der stabilisierten Mischung. Die erfindungsgemäße Mischung wird vorzugsweise wie oben beschrieben hergestellt, das heißt durch Zusammenmischen der Komponenten gegebenenfalls unter Rühren und im allgemeinen bei Raumtemperatur.

Unter Glykolverbindungen werden Oxalkylenglykole, Oxalkylenglykolmonoalkylether und Oxalkylenglykoldialkylether verstanden, wobei Alkyl vorzugsweise ein niedriger Alkylrest und die Oxalkylengruppe vorzugsweise Polyoxethylen und/oder Polyoxpropylen ist. Die erfindungsgemäß zu stabilisierenden Glykolverbindungen sind vorzugsweise solche der nachstehenden Formel

$R^6O(CH_2CHR^7O)_nR^8$

worin $R^6$ für H oder einen Alkylrest mit 1 bis 4 C-Atomen, $R^7$ für H oder $CH_3$, n für eine Zahl von 1 bis 20, vorzugsweise 2 bis 10, und $R^8$ für H oder einen Alkylrest mit 1 bis 4 C-Atomen steht,
wobei die Monoalkylether bevorzugt und die Dialkylether besonders bevorzugt sind. Bezüglich der Oxethylen- und Oxpropylenreste sind die nur Oxethylenreste enthaltenden Glykolverbindungen bevorzugt.

Die erfindungsgemäß stabilisierten Glykolverbindungen weisen eine unerwartet hohe und lang anhaltende thermooxidative Stabilität auch dann auf, wenn sie den harten Bedingungen ausgesetzt sind, die bei gleichzeitiger Einwirkung von Hitze und oxidierenden Komponenten wie Luft oder Sauerstoff vorliegen.

Die Erfindung wird nun an Beispielen und Vergleichsbeispielen noch näher erläutert.

Beispiel 1

0,25 g von der Verbindung der nachstehenden Formel

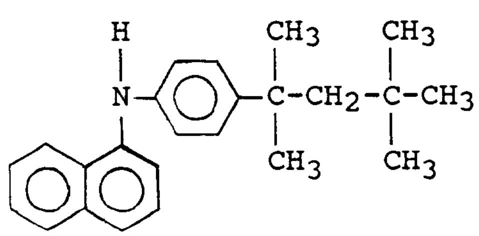

wurden in 50 g einer Ethermischung aus
2 Gew.-% Dimethyltriethylenglykol,
75 Gew.-% Dimethyltetraethylenglykol,
20 Gew.-% Dimethylpentaethylenglykol und
3 Gew.-% Dimethylhexaethylenglykol eingemischt. Die Prüfung der Mischung auf die thermooxidative Stabilität erfolgte nach DIN 51 780 bei einer Temperatur von 120 °C und einem Sauerstoffdruck von 400 kPa (DIN = Deutsche Industrie-Norm). Aus einer Druck-Zeit-Kurve wurde diejenige Zeitdauer in Minuten

festgestellt, die vom Anfangszustand 120 °C und 400 kPa bis zum Zustand 120 °C und 200 kPa verging (das ist 50%ige Druckabnahme, die im wesentlichen aus Sauerstoffverbrauch resultiert). Diese Zeit ist das Maß für die thermooxidativ stabilisierende Wirkung der eingesetzten Verbindungen. Eine stabilisierende Verbindung oder ein Gemisch von solchen Verbindungen ist um so wirksamer je länger diese Zeit ist und umgekehrt.

Vergleichsbeispiel A

Beispiel 1 wurde ohne Einsatz der stabilisierenden organischen Stickstoffverbindung wiederholt.

Beispiel 2

0,5 g von der Verbindung von Beispiel 1 wurden in 50 g Triethylenglykolmonomethylether eingemischt. Die Mischung wurde wie in Beispiel 1 getestet.

Vergleichsbeispiel B

Beispiel 2 wurde ohne Einsatz der stabilisierenden Verbindung wiederholt.

Beispiel 3

0,05 g von der Verbindung von Beispiel 1 wurden in 50 g Diethylenglykol eingemischt. Die Mischung wurde wie in Beispiel 1 getestet.

Vergleichsbeispiel C

Beispiel 3 wurde ohne Einsatz der stabilisierenden Verbindung wiederholt.

In der nachstehenden Tabelle sind die Ergebnisse der Beispiele 1 bis 3 und der Vergleichsbeispiele A bis C zusammengefaßt:

| Beispiel / Vergleichsbeispiel | Zeit in Minuten nach 50%igem Druckabfall gemäß DIN 51 780 |
|---|---|
| 1 / A | 8500 / 190 |
| 2 / B | 12400 / 300 |
| 3 / C | 18000 / 6700 |

Wie die Ergebnisse der Beispiele und der Vergleichsbeispiele zeigen, ist die thermooxidative Stabilität von erfindungsgemäß stabilisierten Glykolverbindungen um ein vielfaches höher als die von nicht stabilisierten.

**Patentansprüche**

**1.** Verfahren zur thermooxidativen Stabilisierung von Glykolverbindungen aus der Gruppe der Oxalkylenglykole und deren Mono- und Dialkylether, dadurch gekennzeichnet, daß den Glykolverbindungen mindestens eine organische Stickstoffverbindung der nachstehenden Formel (1)

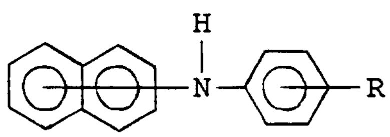

(1)

worin R der nachstehende Alkylrest ist

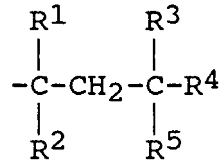

worin $R^1$ bis $R^5$ ein $C_1$ bis $C_4$-Alkyl ist, in einer wirksamen Menge einverleibt wird.

**2.** Verfahren nach Anspruch 1, wobei in Formel (1) $R^1$ bis $R^5$ jeweils Methyl oder Ethyl ist.

**3.** Verfahren nach Anspruch 1 oder 2, wobei die organische(n) Stickstoffverbindung(en) in einer Menge von 0,05 bis 10 Gew.-%, vorzugsweise 0,1 bis 5 Gew.-%, einverleibt wird (werden), Gewichtsprozente bezogen auf die stabilisierte Mischung.

**4.** Verfahren nach einem oder mehreren der Ansprüche 1 bis 3, wobei als Glykolverbindungen solche der nachstehenden Formel eingesetzt werden

$R^6O(CH_2CHR^7O)_nR^8$

worin $R^6$ für H oder einen Alkylrest mit 1 bis 4 C-Atomen, $R^7$ für H oder $CH_3$, n für eine Zahl von 1 bis 20, vorzugsweise 2 bis 10, und $R^8$ für H oder einen Alkylrest mit 1 bis 4 C-Atomen steht.

**5.** Thermooxidativ stabilisierte Mischung bestehend im wesentlichen aus 0,05 bis 10 Gew.-%, vorzugsweise 0,1 bis 5 Gew.-%, von mindestens einer organischen Stickstoffverbindung der im Anspruch 1 angegebenen Formel (1) und mindestens einer Glykolverbindung aus der Gruppe der Oxalkylenglykole und deren Mono- und Dialkylether, die im wesentlichen den Rest auf 100 Gew.-% darstellt, Gewichtsprozente bezogen auf die stabilisierte Mischung.

**6.** Mischung nach Anspruch 5, wobei in Formel (1) $R^1$ bis $R^5$ jeweils Methyl oder Ethyl ist.

**7.** Mischung nach Anspruch 5 oder 6, wobei die Glykolverbindung(en) solche der nachstehenden Formel ist (sind)

$R^6O(CH_2CHR^7O)_nR^8$

worin $R^6$ für H oder einen Alkylrest mit 1 bis 4 C-Atomen, $R^7$ für H oder $CH_3$, n für eine Zahl von 1 bis 20, vorzugsweise 2 bis 10, und $R^8$ für H oder einen Alkylrest mit 1 bis 4 C-Atomen steht.